# EUROPEAN PATENT APPLICATION

(11) **EP 0 860 178 A2**
(43) Date of publication of application: **26.08.1998**
(21) Application number: 98103217.0
(22) Date of filing: 24.02.1998
(51) Int. Cl.: A61N 1/36

(54) **Biomedical device for electrotherapeutic treatments in the psycho/neuro/endrocrino/immunological field**

(30) Priority: 25.02.1997 IT TO970162
(71) Applicant: Sire S.r.l., 50122 Firenze (IT)
(72) Inventor: Iero, Demetrio Paolo, 89100 Reggio Calabria (IT); Di Loreto, Giuseppe, 00040 Roma (IT)
(74) Representative: Jorio, Paolo

(57) **Abstract**

Electronic device comprising a circuit (10) capable of reading, in response to an activation signal (40), a stored waveform in order to generate an output signal representative of this waveform, which signal is transmitted to a voltage amplifier (14) which generates a variable-voltage output signal (14u) representative of the stored waveform. The variable-voltage signal is applied to a transducer (7) constituting at least one conductive electrode (24) applied to an individual, preferably to areas on the back of the head corresponding to cutaneous projections of deep areas of the brain, in order to apply an electrical or electromagnetic stimulus to the nervous system of the individual, in response to which the brain emits infinitesimal quantities of neurotransmitters which act upon the endocrine organs of the human body which, in turn, release hormones to stimulate the immune system and bring about a process of the feedback type.

## Description

The present invention relates to an electronic device to provide electronic assistance to biomedical treatments in the psycho/neuro/endocrino/immunological field.

The area of application of the present invention is medicine in the broad sense (including sports medicine and aesthetic medicine) and/or psychology, in which disorders of the human body or mind are usually treated with drugs and/or psychiatric therapy. One example of the field of application of the present device is the treatment of depression or states of anxiety, mental disorders which are usually treated with anti-depressants or anxiolytics combined with psychiatric therapy.

Prior art electronic devices which can be used in the above-stated applications are generally known as "BIOFEEDBACK" devices, and are capable of detecting physiological parameters (for example temperature, sweating, heart rate, skin pH, blood pressure etc.) which are indicative of the psychological or physical state of the individual, in order to provide the individual himself directly with an immediate indication of his physical and/or psychological state of health. On the basis of the detected state of health, the individual immediately becomes aware of possible problems or pathological states as they occur and teaches himself to overcome the associated conditions. Such appliances are clearly of the entirely passive type in that they are only capable of measuring and revealing a state of health.

The object of the present invention is to produce an electronic appliance of the active type for treatment of illnesses of the human body and mind.

The above object is achieved according to the invention by a device of the type described in Claim 1.

The invention will now be described with reference to the attached drawings which show a preferred, non-limiting embodiment thereof, in which drawings:
Figure 1 shows a simplified electrical circuit diagram of an electronic device produced in accordance with present invention; and
Figure 2 shows an electrical signal generated by the device of Figure 1.

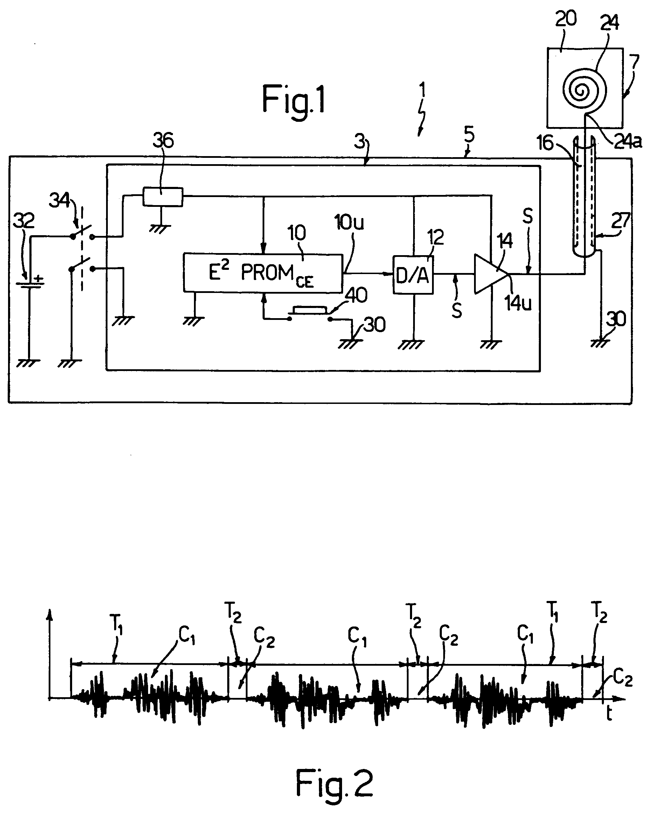

Reference numeral 1 in Figure 1 denotes the electronic device, taken as a whole, to provide electronic assistance to biomedical treatments in the psycho/neuro/endocrino/immunological field, the device being referred to below for of the sake simplicity as the device 1. The device 1 comprises an electronic circuit 3 advantageously accommodated in an impact-resistant housing 5 (for example a plastic housing) and a transducer 7 which is outside the housing 5 and connected to the circuit 3.

The circuit 3 principally comprises an integrated circuit 10 to store waveforms, in particular an EEPROM integrated circuit constituting a digital waveform recorder comprising a high capacity memory (for example a 4 Mbit capacity memory) containing digital data representative of stored waveforms. The stored digital data can be transferred, on receipt of an ENABLE signal, to an output 10u of the integrated circuit 10 towards a digital/analogue converter 12, the input of which is connected to the output 10u, said converter having an output connected to an input of a voltage amplifying circuit 14, which has a pass band within the low-frequency range (for example between 0.5 Hz and 50 kHz) and a low-power output (for example a power of 0.8 W). The voltage amplifying circuit 14 has an output 14u which communicates via an electrical conductor 16 with a transducer 7. Specifically, the transducer 7 comprises a generally flat insulating substrate 20 made from an insulating material, for example glass fibre-reinforced plastic or Vetronite, on which is applied a conductive metal track 24 (for example made from copper) which is electrically connected to the conductor 16.

The track 24 is in the form of a clockwise or anti-clockwise spiral (for example comprising 5 turns) having an external end 24a connected to the electrical conductor 16. The flat substrate 20 can be of the non-insulating type but can be covered with an insulating material, for example glass fibre reinforced polyethylene etc.

The electrical conductor 16, which is flexible and extends outside the housing 5, is furthermore provided over its entire length with electromagnetic shielding 27, advantageously constituted by a metallic sleeve connected to a reference potential 30 (earth).

The electronic circuit 3 is powered by an external source of direct current 32 (conveniently constituted by a 9 V battery), which can be connected to the circuit 3 by means of a double switch 34 and is capable of powering a voltage-reducing integrated circuit 36 (for example a circuit capable of reducing the voltage of 9 V from the battery 32 to a stabilised voltage of a few volts) used for powering the integrated circuit 10 and, optionally, the converter 12 and amplifier 14.

An example of a waveform stored within the integrated circuit 10 is shown in Figure 2.

The waveform shown in Figure 2 comprises first signal cycles C₁ having a preset duration T₁ (for example a duration of 2-3 seconds) alternating with pause cycles C₂ having a preset duration T₂ (for example 1 second), during which the stored signal is zero. The duration T₁ of the signal cycles is greater than the duration T₂ of the pause cycles.

The waveform included in the signal cycle C₁ generically represents a low-frequency type signal, a series of low-frequency impulses or the digitisation of a voice signal corresponding to a phrase spoken in any language. The signals of successive cycles C₁ can be identical or can have different waveforms.

In use, the transducer 7 is applied to the body of an individual (not shown) with the conductive track 24 being arranged in direct contact with the skin of the individual or with the conductive track 24 being arranged close to the skin of the individual and constituting electrical insulation from the skin itself by means of a layer of insulating material (not shown) placed on top of the conductive track 24.

The transducer 7 is preferably arranged close to the back of the head in specific areas corresponding to cutaneous projections of deep areas of the brain, in particular in the vicinity of the thalamic areas of the brain. The switch 34 is then closed, activating the power supply to the entire circuit 3; an ENABLE command is then transmitted to the integrated circuit 10, the enable signal, for example, possibly being produced by closing a push-button switch 40 arranged between an enable input CE of the integrated circuit 10 and the reference potential 30. The stored waveform is then taken from the memory of the integrated circuit 10 and passed to the converter 12 which generates an output voltage signal S which reproduces the stored waveform. The signal S is then amplified by the amplifier 14 and applied to the transducer 7. The waveform is repeated in a known manner for as long as the circuit 3 is active. The voltage on the transducer 7 is, however, variable, reproducing the stored waveform as it changes over time; this variable voltage is applied directly to the skin of the individual (in the case of direct contact between the transducer and human body) or produces an electrical field applied to the human body (in the presence of insulation between the transducer and human body). In either case, the nervous system of the individual is stimulated (NEUROLOGICAL treatment) and the stimulus is transmitted to the brain. It is hypothesised that, in response to such an electrical stimulus, the brain of the individual emits infinitesimal quantities of neurotransmitters which act upon the endocrine organs (ENDOCRINE treatment) of the human body (thyroid, adrenal, thymus glands etc.) which, in turn, release hormones which stimulate the immune system (IMMUNOLOGICAL treatment). The stimulation and consequent strengthening of the immune system bring about an improvement in the general state of health of the individual, both physical and psychological (PSYCHOLOGICAL treatment); this improvement in general condition, combined with other pharmacological or psychological therapy, contributes towards the elimination or reduction of the psychophysical problems of the individual (feedback process). The synergistic action of such effects, which are precisely of a PSYCHOLOGICAL, NEUROLOGICAL, ENDOCRINAL and IMMUNOLOGICAL nature, justifies the name of the device itself.

It is clear from the above description how the electronic device according to the invention provides active treatment of illnesses of the human body and mind.

Finally, it is clear that the electronic device described can be modified or varied without extending it beyond the scope of protection of the present invention. For example, the converter 12 and amplifier 14 could also be combined into a single integrated circuit.

## Claims

1. Electronic device to provide electronic assistance to biomedical treatments in the psycho/neuro/endocrino/immunological field, characterised in that it comprises:
- storage means (10) capable of storing at least one waveform of a low-frequency signal;
- reproduction means (10) capable of reading, in response to an activation signal (40), the stored waveform to generate an output signal representative of the stored waveform;
- amplification means (14) receiving said signal representative of the stored waveform as the input and generating a variable-voltage output signal (14u) representative of the stored waveform; and
- transducer means (7) constituting at least one conductive electrode (24) communicating (16) with the output of said amplification means (4) in order to receive said variable-voltage signal; said transducer means (7) being capable of being applied to an individual with said electrode (24) arranged at least close to a part of the body of the individual, in particular to the back of the head on specific areas of skin corresponding to cutaneous projections of deep areas of the brain, in order to apply an electrical or electromagnetic stimulus to the nervous system of the individual, in response to which the brain emits infinitesimal quantities of neurotransmitters which act upon the endocrine organs of the body which, in turn, release hormones to stimulate the immune system.

2. Device according to Claim 1, characterised in that said waveform comprises first signal cycles (C₁) having a preset duration (T₁) alternating with second pause cycles (C₂) having a preset duration (T₂), during which the stored signal is substantially zero.

3. Device according to Claim 2, characterised in that the duration (T₁) of the first cycles (C₁) is greater than the duration (T₂) of the pause cycles (C₂).

4. Device according to Claim 2 or 3, characterised in that the waveform of the first cycles (C₁) comprises a series of low-frequency impulses.

5. Device according to Claim 2 or 3, characterised in that the waveform of the first cycles (C₁) relates to the digitisation of a voice signal.

6. Device according to any of the preceding claims, characterised in that said transducer means (7) comprise an insulating substrate (20) on which is arranged a conductive metallic track (24) constituting said electrode; said track (24) being in the form of a spiral with an external end (24a) connected to the output (14u) of said amplification means (14).
